(19) Europäisches Patentamt
European Patent Office
Office européen des brevets

(11) Publication number: **0 243 126 B1**

(12) **EUROPEAN PATENT SPECIFICATION**

(45) Date of publication of patent specification: **12.08.92**

(51) Int. Cl.[5]: **C12Q 1/28**, C12Q 1/26

(21) Application number: **87303433.4**

(22) Date of filing: **16.04.87**

# (54) Method of measuring hydrogen peroxide or of measuring an enzyme or biochemical substrate liberating the peroxide.

(30) Priority: **19.04.86 JP 89203/86**
**19.04.86 JP 89204/86**

(43) Date of publication of application:
**28.10.87 Bulletin 87/44**

(45) Publication of the grant of the patent:
**12.08.92 Bulletin 92/33**

(84) Designated Contracting States:
**CH DE FR GB IT LI**

(56) References cited:
**EP-A- 0 033 539**
**EP-A- 0 123 902**
**DE-A- 3 213 786**
**US-A- 4 378 429**
**EP-A- 0 054 358**
**EP-A- 0 148 275**
**GB-A- 2 095 401**
**US-A- 4 427 770**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Uno, Shizuo**
**2-116-2, Fujigaoka**
**Kasugai-shi Aichi-ken(JP)**
Inventor: **Kurono, Masayasu**
**3-6-7, Sasao-nishi Toin-cho**
**Inabe-gun Mie-ken(JP)**
Inventor: **Mikamo, Mari**
**6-3-17, Fujiyamadai**
**Kasugai-shi Aichi-ken(JP)**
Inventor: **Ito, Shinobu**
**3-50-3, Gunsui-cho Mizuho-ku**
**Nagoya-shi Aichi-ken(JP)**
Inventor: **Sawai, Kiichi**
**1-36-14, Ninomiya**
**Funabashi-shi Chiba-ken(JP)**

(74) Representative: **Diamond, Bryan Clive et al**
**Gee & Co., Chancery House, Chancery Lane**
**London WC2A 1OU(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a highly sensitive method of measuring hydrogen peroxide in a sample as well as a method of quantitatively measuring enzymes and biochemical substances which are enzyme substrates.

In the field of clinical examinations for measuring various biochemical substances and enzymes in a sample obtained from a living body, namely a serum, urine or the like, quantitative methods which utilize a hydrogen peroxide forming reaction have been widely carried out, as shown below.

Glucose (Glu)

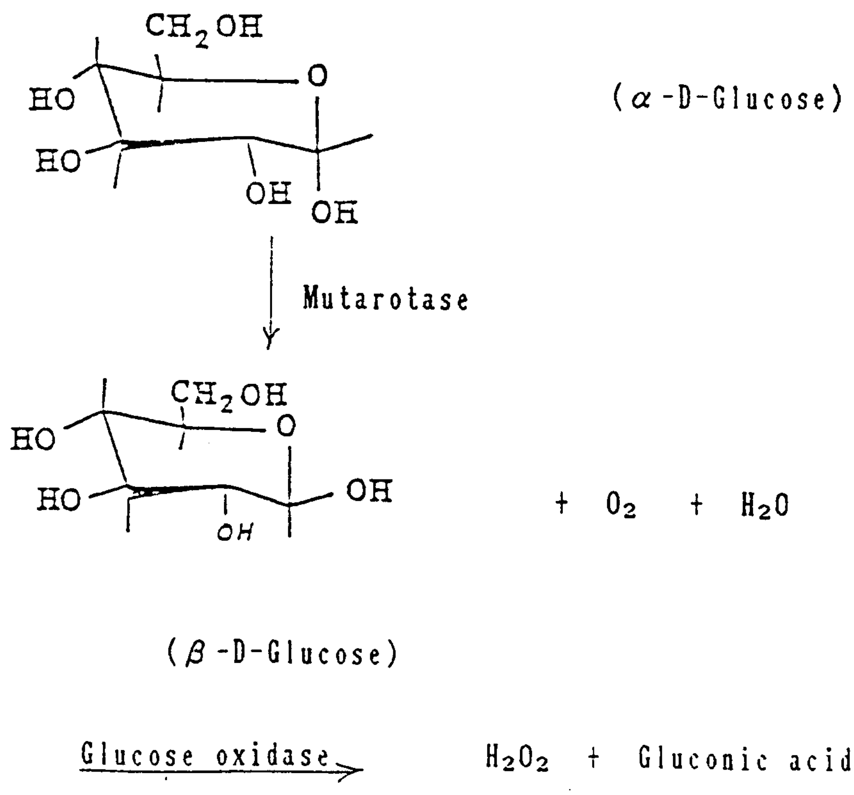

Uric Acid (UA)

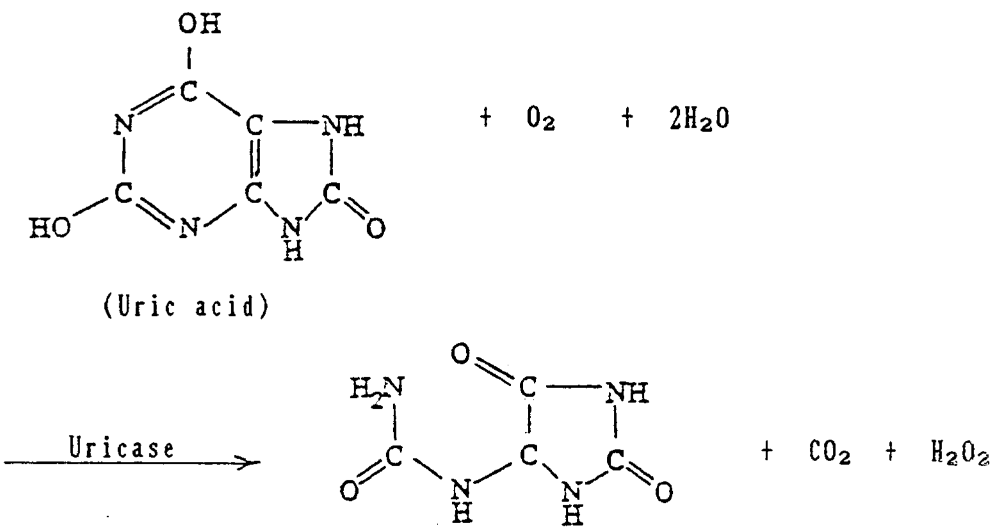


(Uric acid)

(Allantoin)

Non-esterified Fatty acid (NEFA)

$$RCOOH + ATP + CoA \xrightarrow{ACS} RCO\text{-}CoA\text{-}AMP + PPi$$

(PPi : Pyrophosphoric acid)

$$AMP + ATP \xrightarrow{MK} 2ADP$$

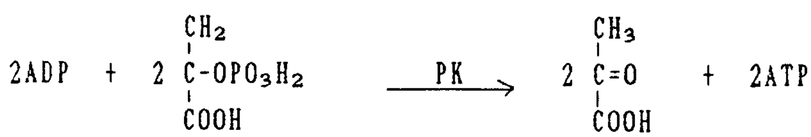


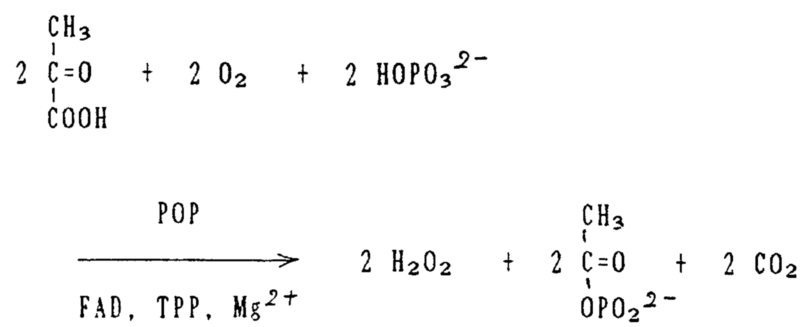


(Acetylphosphoric acid)

In the above formulae, the symbols mean as follows.

ACS : Acyl-CoA synthetase
MK : Myokinase
PK : Pyruvate kinase
POP : Pyruvate oxidase

TPP : Thiamine pyrophosphate

## Cholesterol

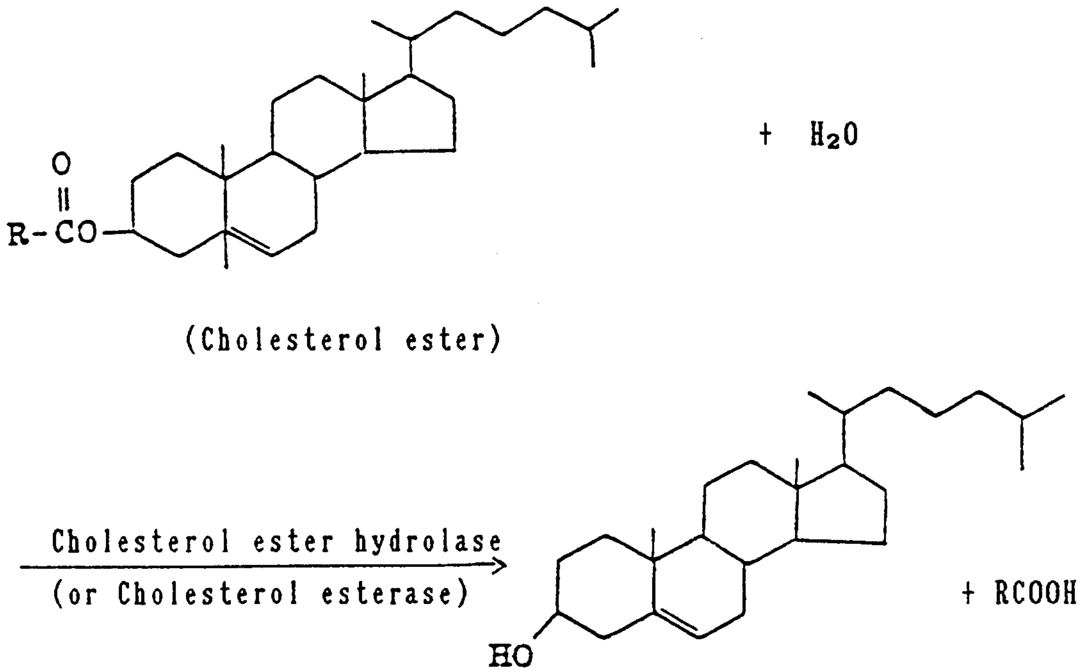


+ $H_2O$

(Cholesterol ester)

+ RCOOH

(Cholesterol)

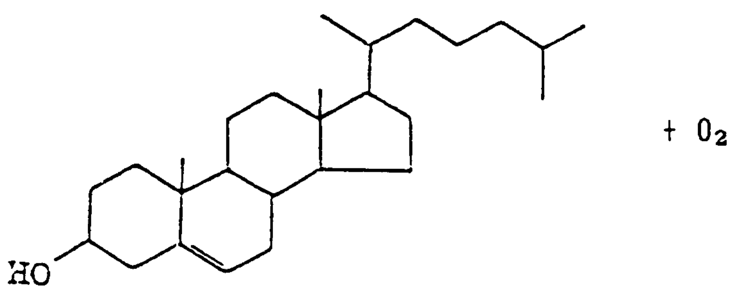

+ $O_2$

(Cholesterol)

Cholesterol oxidase

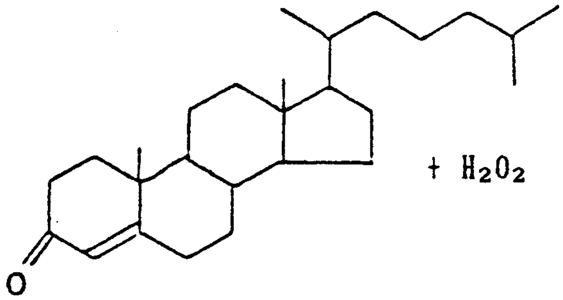

+ $H_2O_2$

($\Delta^4$-Cholestenone)

Serum Transaminase (GOT, GPT)

GOT (Glutamic oxaloacetic transaminase) :

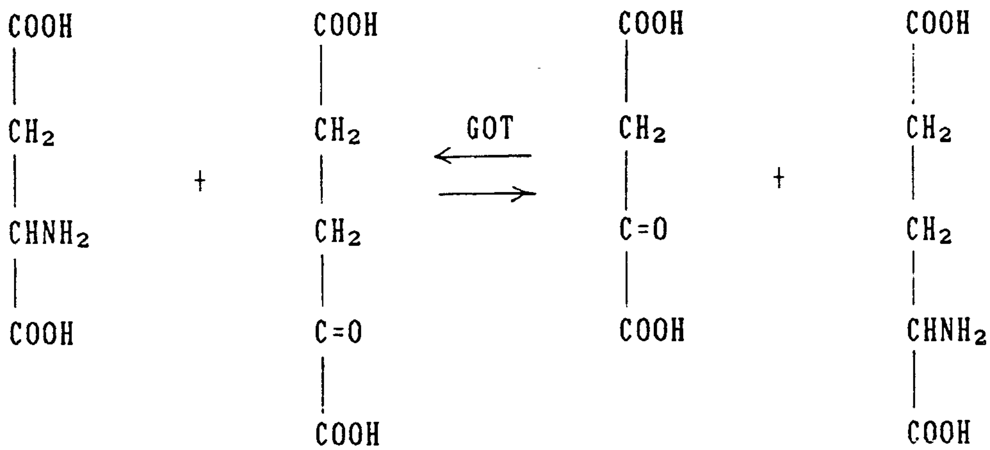


(L-Aspartate) ($\alpha$-Oxoglutarate) (Oxaloacetate) (Glutamate)

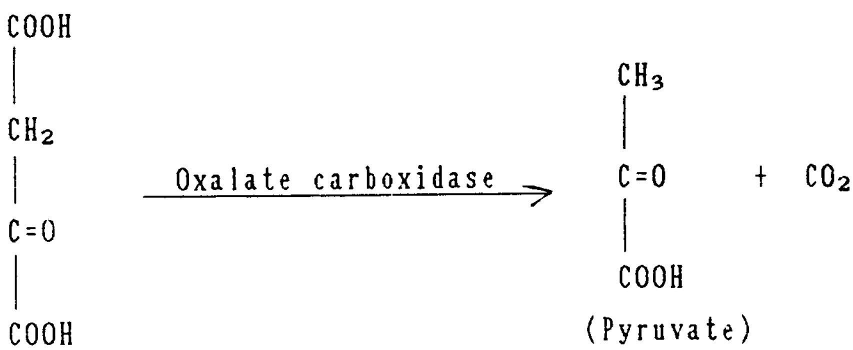


(Pyruvate)

GPT (Glutamic pyruvic transaminase) :

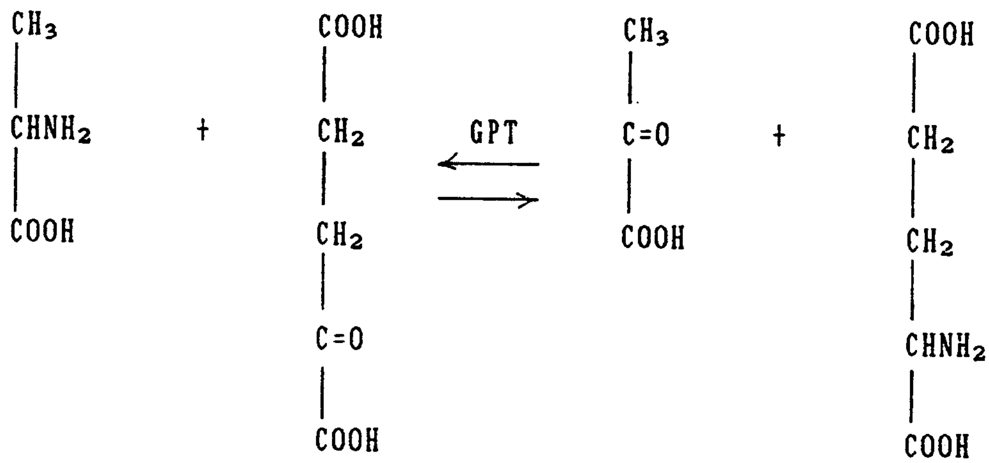


(L-Alanine) (α-Oxoglutarate) (Pyruvate) (Glutamate)

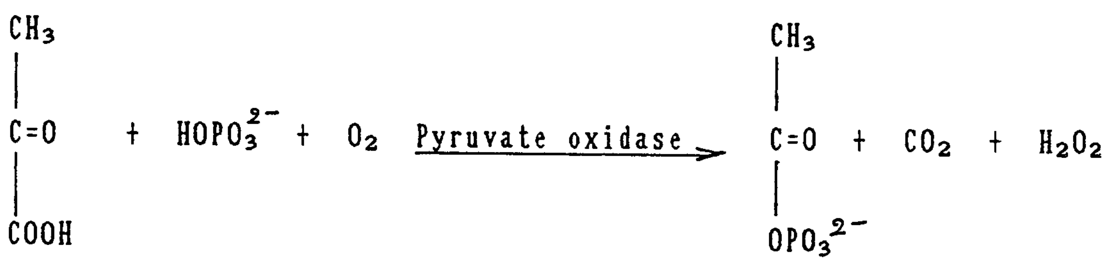


(Acethylphosphoric acid)

## Phosphoric Lipids (PL)

Phosphoric lipid {Lecithin, Sphingomyelin, Lysolecithin} + $H_2O$ Phospholipase D →

$[HO-CH_2-CH_2-N(CH_3)_3]^+OH^-$ + {Phosphatidic acid, N-Acylsphingocylphosphate, Lysophosphatidic acid}

(Choline)

$$[HO-CH_2-CH_2-N(CH_3)_3]^{+}OH^{-} + 2\ O_2$$

(Choline)

$$\xrightarrow{\text{Choline oxidase}} {}^{-}OOC-CH_2-N^{+}(CH_3)_3 + 2 \cdot H_2O_2$$

(Betaine)

<u>Triglyceride (TG)</u>

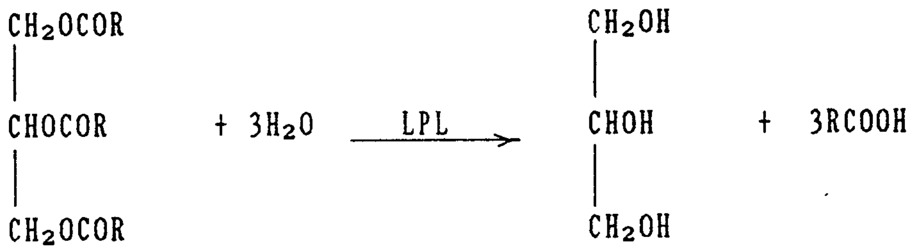


(Triglyceride) (Glycerine)

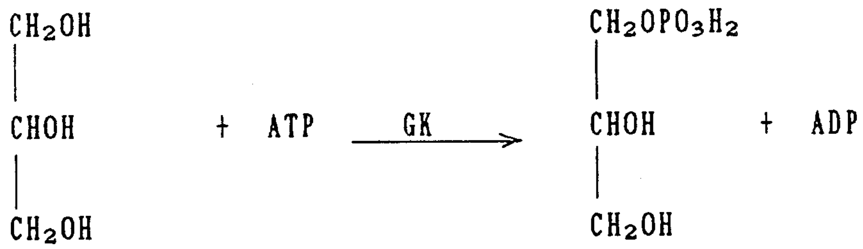


(Glycerine-3-phosphate)

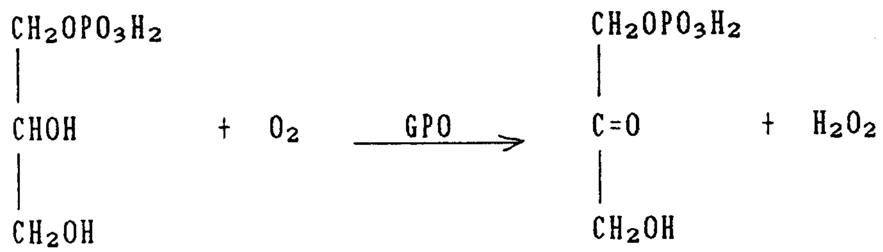


(Dihydroxyacetophosphate)

In the above formulae, symbols mean as follows.

| | |
|---|---|
| LPL : | Lipoprotein lipase |
| GK : | Glycerokinase |
| GPO : | Glycerol-3-phosphate oxidase |

Cholinesterase (ChE)

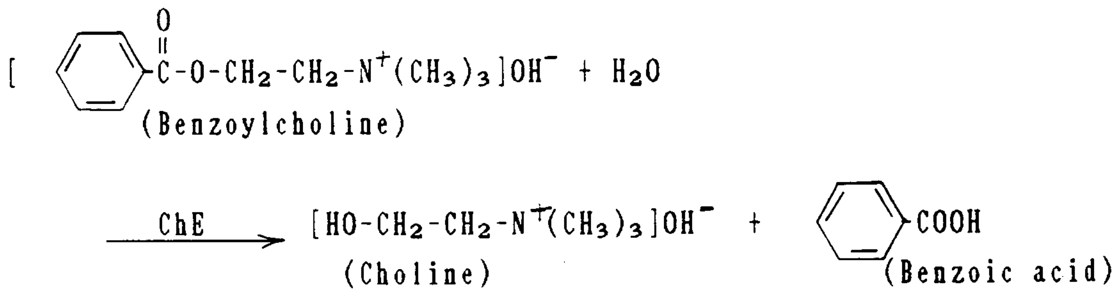


(Benzoylcholine)

(Choline)

(Benzoic acid)

$$[HO\text{-}CH_2\text{-}CH_2\text{-}N^+(CH_3)_3]OH^- + 2\ O_2 \xrightarrow{\text{Choline oxidase}} 2\ H_2O_2 + {}^-OOC\text{-}CH_2\text{-}N^+(CH_3)_3$$

(Betaine)

The hydrogen peroxide, biochemical substance or enzyme has been quantitatively measured by subjecting the hydrogen peroxide formed in each of said reactions to a coloring reaction with use of peroxidase (POD) or catalase and measuring the resulting color tone or intensity, as stated below.

Methods using POD

The principle of the methods may be shown by the following reaction:

$H_2O_2 + DH_2 \rightarrow D + 2H_2O$ ($DH_2$ : Potential dye material)

Methods using catalase

There are methods, for instance, one wherein methanol is oxidized with hydrogen peroxide and then adding acetylacetone and an ammonium salt to the resulting formaldehyde to develop a color according to Hantz's reaction. This method can be shown by the reaction formulae given below:

$$H_2O_2 + CH_3OH \longrightarrow HCHO + 2H_2O$$

$$HCHO + 2\ CH_3COCH_2COCH_2 + NH_3$$

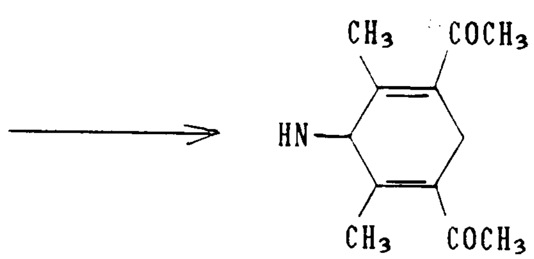

(measured at 410nm)

In these methods, the latter methods using catalase require a relative long period of time and are

difficult to adapt to an automatic measuring apparatus, while the former methods using POD have widely been employed in the field of clinical examinations. Various methods using POD have been proposed, which are classified generally into following 4 types.

a) Methods using o-dianidine or o-tolidine:

These types of methods have not been utilized now, since the dyes formed are not so stable and have a possible risk of generating cancer.

b) Methods using 4-aminoantipyrine and a phenol:

These types of methods were firstly reported by Trinder in 1969 and have been widely employed. According to the methods, a reddish dye is formed by the reaction of

$$2\ H_2O_2 + \text{4-aminoantipyrine} + \text{phenol compound} \xrightarrow{POD} \text{a reddish quinonic dye} + 4\ H_2O$$

and the color tone of the dye is measured at a wave-length of about 500 nm. As the phenols, p-hydroxy benzoic acid, p-chlorophenol, 2,4-dichlorophenol, 4-6-dichloro-o-cresol, 2,4-dibromophenol, 4,6-dibromo-o-cresol and the like have been employed.

c) Methods using 4-aminoantipyrine and an aniline:

These types of methods have been developed based on said type b method. As the anilines, dimethylaniline, diethylaniline and the like have been employed and the color tone or intensity of the dye formed is measured in a higher wave-length range of 400 to 600 nm.

d) Others:

As other types of methods, the following have also been proposed:

i)

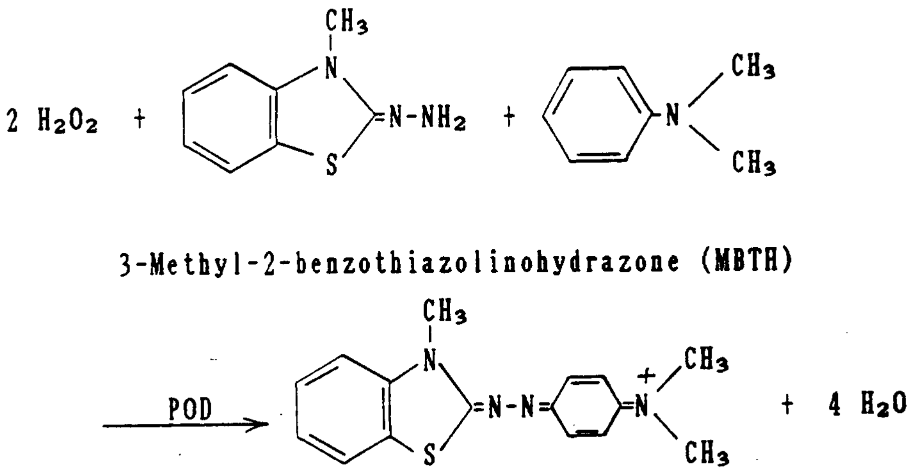

3-Methyl-2-benzothiazolinohydrazone (MBTH)

Indamine dye (to be measured at 500nm)

ii)

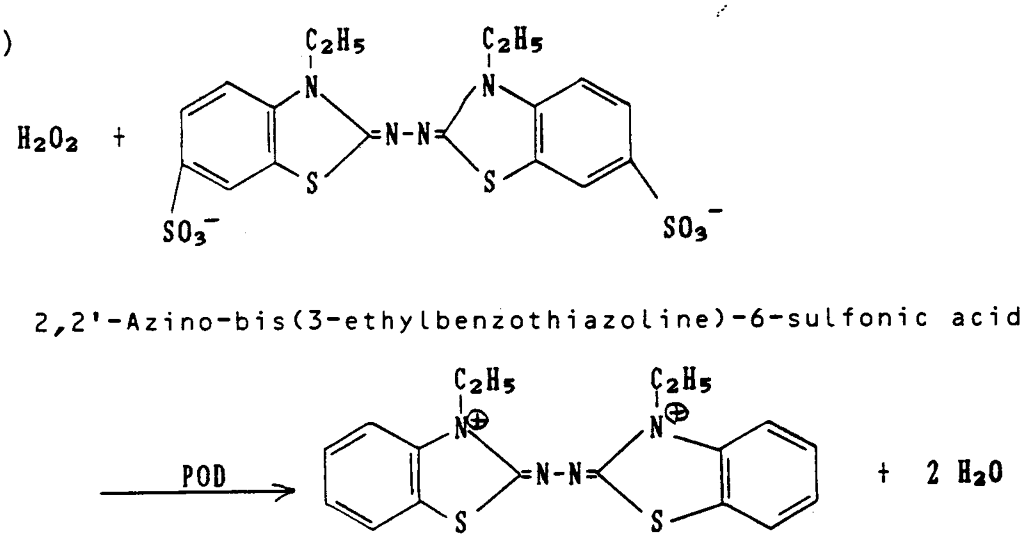

2,2'-Azino-bis(3-ethylbenzothiazoline)-6-sulfonic acid

At present, the above four types of methods using POD as the oxidase have been actually employed in the field of clinical examinations for leading hydrogen peroxide formed by the enzymatic reaction between the biochemical substance and enzyme, in a sample, or hydrogen peroxide per se in another sample into the coloring reaction and quantitatively determining an amount of the biochemical substance, enzyme or hydrogen peroxide, based on the measurement of the color tone or intensity of the formed dye. Although, in the field of clinical examinations, a serum or urine is generally selected as a test sample for quantitatively determining such a substance, these conventional methods are effective when a normal sample is used for the test. However, if the test sample is a possible abnormal one of a hemolytic, chylous, icteric or the like sample, each of the conventional methods shows a disadvantage that each of haemoglobin, chyme, bilirubin and the like acts as a disturbing material to cause an error in measurement, since each maximum absorption of the materials overlaps with or near the measuring wave-length (less than 600nm) for the dye to be formed through the conventional methods, as follows.

Haemoglobin : having dual maximum absorptions at 430nm and range of 530 to 570nm,
Chyme : absorbing over a wide wave-length range and showing a higher absorbancy, as a measurement wave-length will become shorter, and
Bilirubin : having a maximum absorption at 465nm.

Further, a serum or urine as the test sample may include ascorbic acid which is one of various drugs employed widely and has been known to cause a negative error in measuring value, if it is present in the sample in a relatively high concentration (about 5mg/dl or more). For decreasing or inhibiting the influence

of ascorbic acid, there is required an additional operation of increasing the concentration of 4-aminoantipyrine, decreasing the concentration of POD or oxidizing ascorbic acid by addition of $Cu^{2+}$ ion, periodic acid or ascorbate oxidase.

An object of the invention is to provide a highly sensitive method of measuring hydrogen peroxide in a sample, even if the sample is not a normal one.

Another object of the invention is to provide a highly sensitive method of measuring biochemical substances and enzymes in a sample, even if the sample is not a normal one.

EP-A-0033539 describes a method of measuring hydrogen peroxide; by reacting (a) a phenolic compound such as phenol or naphthol, (b) a novel aminoantipyridine compound which can be a diamine compound, and (c) a peroxidase enzyme and measuring with a photometer the color of the resultant solution, e.g. at a wavelength of 546 nm (Ex. 6). The present invention provides measurement with decreased interference from other substances present in the sample.

According to the invention, we provide a method of quantitatively measuring hydrogen peroxide in a sample, which comprises adding to the sample: (a) a phenol or naphthol compound of the formula

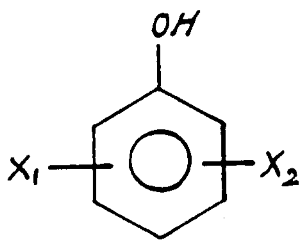

or

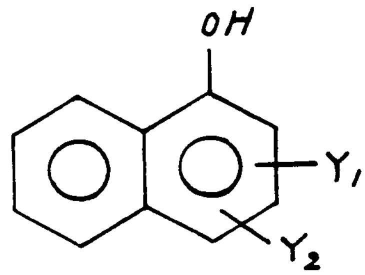

wherein $X_1$ and $X_2$ are each a hydrogen atom, halogen atom, alkyl group or nitro radical, and $Y_1$ and $Y_2$ are each a hydrogen atom, halogen atom, nitro radical, sulfo radical or acyl group, (b) p-diethylaminoaniline and (c) an oxidase, to cause a reaction and form a quinonic dye, and measuring the intensity of the color of the resulting solution to determine the amount of the hydrogen peroxide.

THe invention also includes a method of quantitatively measuring biochemical substances and enzymes in a sample obtained from a living body, which comprises reacting the sample with the enzyme to liberate hydrogen peroxide, adding the reagents (a), (b) and (c) as defined above to cause a reaction with the liberated hydrogen peroxide and form a quinonic dye, and measuring the color tone of the resulting solution to determine the amount of biochemical substance or enzyme in the sample.

As examples of the component (a) phenol, p-hydroxybenzoic acid, p-chlorophenol, 2,4-dichlorophenol, 4,6-dichloro-o-cresol, 2,4-dibromophenol, 4,6-dibromo-o-cresol may be listed as phenols, and α-naphthol, 1-naphthol-2-sulfonic acid may be listed as naphthols.

As the oxidase (c), peroxidase (POD) or catalase may be employed; it is preferred to use POD, most preferably vegetable POD extracted from a horseradish, garden radish or the like and refined, but one extracted from animal or bacteria may also be used if it has been sufficiently refined.

A main reaction occuring in the method of the invention may be shown as follows, wherein phenol, α-naphthol or 1-naphthol-2-sulfonic acid is used as the phenol or naphthol compound and POD is used as the oxidase.

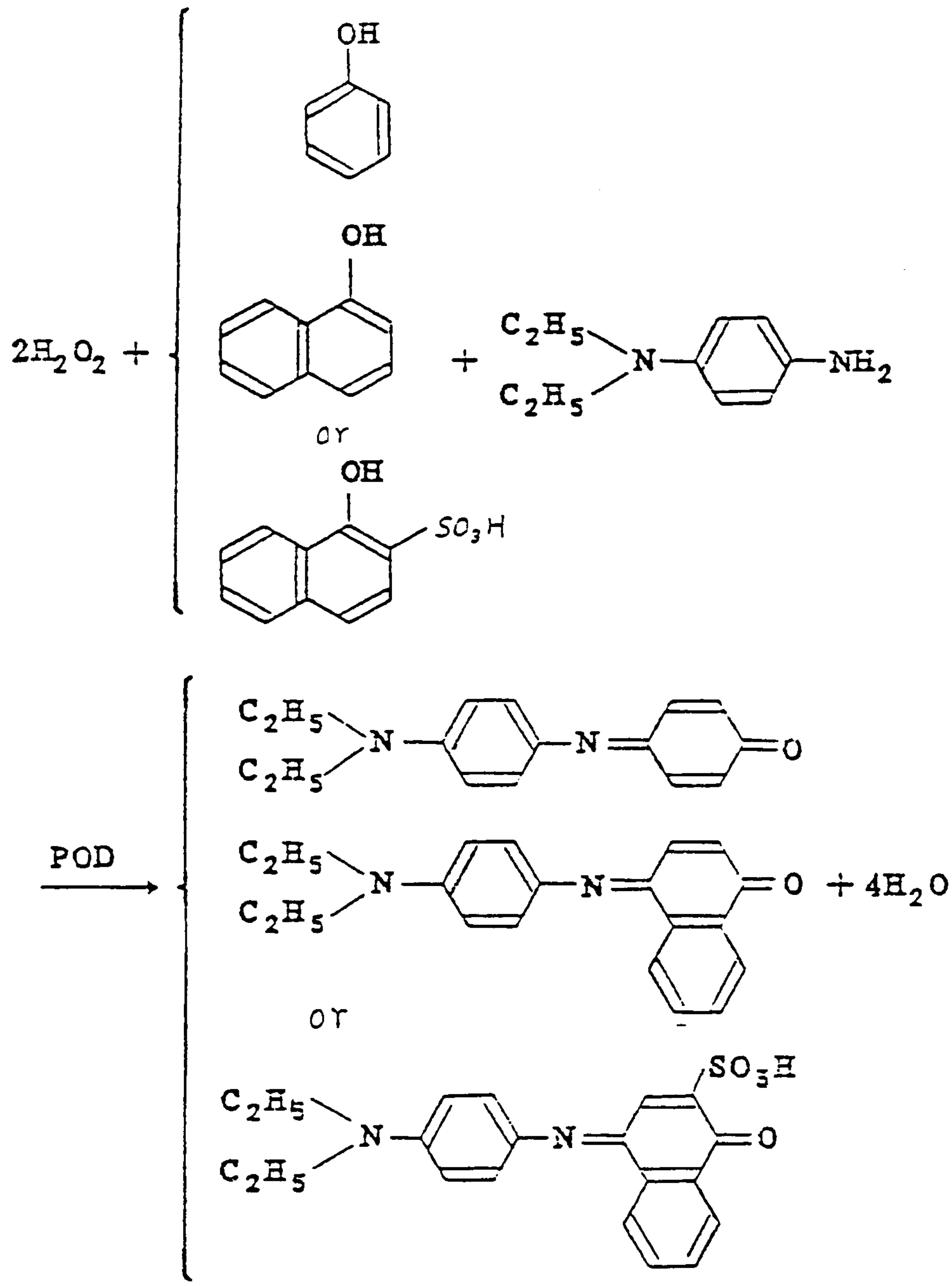


Each of the resulting compounds is a quinonic dye having a maximum absorption at or near 650nm.

The invention will now be explained with reference to test Examples and measuring Examples, which may refer to the drawings, in which

Fig. 1 is a graph showing extinction spectrum to check a relation between absorbency and a measurement wave-length, on each of a test serum, standard and control samples for measuring uric acid, in accordance with the invention;

Figs. 2 to 4 are graphs showing the influences of bilirubin, intralipid and hemoglobin, respectively on a measured value;

Fig. 5 is a graph showing course of the reaction, after addition of a second reagent, in the measurement of uric acid;

Fig. 6 is a graph showing the relation between oncentration of uric acid and absorbancy of the solution, which may be used as a calibration curve; and

Fig. 7 is a graph showing the correlation between the method of the invention and a conventional method, in the measurement of uric acid.

Example 1 (Quantitative measurement of hydrogen peroxide)

[1] Reagents:

a) Phosphate buffer: Aqueous solution prepared by mixing 100 mmol/l sodium hydrogen phosphate with 100 mmol/1 potassium dihydrogen phosphate and adjusting pH of the mixture to 7.0.
b) Naphthol compound: Aqueous solution of 4 mmol/l potassium 1-naphthol-2-sulfonate.
c) Aqueous solution of 3.5 mmol/l p-aminodiethylaniline dihydrochloride.
d) Peroxidase: A commercially available peroxidase (Grade II) sold by Boehringer Mannheim GmbH, West Germany was diluted with distilled water to prepare a 0.3 unit/ml solution.

[II] Measuring operations

a) A sample solution (T) containing hydrogen peroxide, a standard solution (St) containing hydrogen peroxide of 50 μM/l and refined water as control were each taken in 50 μl, portions, and each solution was charged into a separate test tube.
b) To each of the test tubes, 2.0 ml of the phosphate buffer, 0.15 ml of the naphthol compound solution and 0.15 ml of the diamine compound solution were added and mixed.
c) Each of the mixtures was heated and kept at 37° C for 5 minutes.
d) The absorbancy of the mixtures containing the sample and that containing standard solution was measured at 675 nm, while comparing each with the absorbancy of the control mixture, to obtain measured values of Eo(T) and Eo(St), respectively.
e) 0.1 ml of the POD solution was further added to each of the test tubes and mixed with the solution therein.
f) The resulting mixture was heated and kept at 37° C for 5 minutes.
g) The absorbancy of the mixture containing the sample and that containing standard solution was measured at 675 nm, while comparing each with the absorbancy of the control mixture, to obtain measured values of Ef(T) and Ef(St), respectively.

[III] Quantitative calculation:

The solution C (μM/l) of hydrogen peroxide in the sample was calculated in accordance with the following equation:

$$C = \frac{Ef(T) - [Eo(T) \times (2.35/2.45)]}{Ef(St) - [Eo(St) \times (2.35/2.45)]} \times 50$$

[IV] Results:

It was found that concentrations of hydrogen peroxide in various sample solutions, which varied in a range of 0 to 200 μM/l, can be quantitatively measured.

Example 2 (Quantitative measurement of uric acid)

[I] Reagents:

a) First reagent

This reagent was prepared by dissolving 1 mg of potassium 1-naphthol-2-sulfonate, 35 units of peroxidase and 40 units of ascorbate oxidase, into 20 ml of 50 mmol/l phosphate buffer (pH 5.9) which contained 1% of polyoxyethylene octylphenyl ether. This reagent can be effectively used over several days, if it is stored under ice cooling.

b) Second reagent

This reagent was prepared by dissolving 3 mg of p-aminodiethylaniline dihydrochloride and 40 units of uricase into 10 ml of 50 mmol/l phosphate buffer (pH 5.9). This reagent can also be stably stored over several days, if it is kept under dark and ice cooling conditions.

[II] Measuring operations:

a) A sample of serum from human blood ($T_1$), a standard solution containing uric acid of 10 mg/d1 ($St_1$) and refined water as control were taken each in a volume of 50 u1, and each solution was charged into a separate test tube.
b) To each of the test tubes, 1.75 ml of the first reagent were added and mixed, and then each mixture was heated to and kept at 37° C for 5 minutes.
c) The absorbancy of the mixture containing the sample and that containing the standard solution was measured at 650 nm, while comparing each with the absorbancy of the control mixture, to obtain measured values of $EO(T_1)$ and $EO(St_1)$, respectively.
d) To each of the test tubes, 0.25 ml of the second reagent were further added and mixed, and then the mixtures were heated to and kept at 37° C for 5 minutes.
e) The absorbancy of the mixture containing the sample and that containing standard solution was measured at 650 nm, while comparing each with the absorbancy of the control mixture, to obtain measured values of $Ef(T_1)$ and $Ef(St_1)$, respectively,.

[III] Quantitative calculation:

The concentration $C_1$ (mg/d1) of uric acid in the sample was calculated in accordance with the following equation:

$$C = \frac{Ef(T_1) - [Eo(T_1) \times (1.8/2.05)]}{Ef(St_1) - [Eo(St_1) \times (1.8/2.05)]} \times 10$$

Test Example 1 (Extinction spectrum on measurement of uric acid)

The uric acid measuring operations described in Example 2 were carried out by variously changing the measuring wave-length in a range of 500 to 800 nm to check a relation between the measuring wave-length and absorbancy and obtain results as shown in Fig. 1. As is seen from the figure, both the test serum sample and standard sample have a maximum absorption near 650 nm and there is no deviation in both extinction spectra due to the wave-length used for the measuring.

Test Example 2 (Influences of disturbing substances)

To check the influence of bilirubin, chyme and hemoglobin upon the measured value of uric acid in the method as described in Example 2, bilirubin, intralipid (a lipid emulsifier for injections) or hemoglobin (a supernatant prepared by physically breaking washed red blood corpsucles and centrifuging them) was added to a normal serum sample, and the uric acid measuring operations as disclosed in Example 2 were carried out to obtain the results given in Figs. 2 to 4. As is evident from the figures, the subtances regarded in conventional methods as causes of introducing an error in measurement exert almost no influence on the measured value.

Test Example 3 (Required period of time for reactions)

In Example 2, each period of time for the reaction after adding the first reagent and for the reaction after adding the second reagent was set at 5 minutes, but the required time may be made shorter, as stated below.

The first reagent is added to eliminate the influence of ascorbic acid which possibly is present in the sample serum and to remove a possible substance or substances which behaves similarly to hydrogen peroxide. The reaction with the second reagent which is to be added to form hydrogen peroxide from the uric acid and to develop the color in the reaction solution finishes 3 to 4 minutes after adding the second reagent, as shown in Fig. 5. This fact of short period of time required for the reaction means the method of the invention may be utilized in an automatic analyzer.

Test Example 4 (Calibration curve for measuring uric acid)

The relation between concentration of uric acid and absorbancy was checked by carrying out the method described in Example 2, to find that there were a good linear relation in the uric acid concentration range of 0 to 80 mg/d1, as shown in Fig. 6. Therefore, the graph shown in this figure can be employed as a calibration curve for quantitatively measuring uric acid.

Test Example 5 (Reproducibility and recovery in addition)

Reproducibility and recovery in addition of the method of the invention as described in Example 2 were measured, to obtain the results shown in following Tables 1 and 2. As seen from the Tables, the reproducibility shows the good value of CV = 0.52 to 0.80 (%) and the recovery in addition shows the value of about 100%. Thus the accuracy of the invention method is equal to or superior than that of the conventional methods.

Table 1

| | Standard solution | Serum 1 | Serum 2 |
|---|---|---|---|
| | (mg/dl) | | |
| | 9.9 | 9.2 | 16.5 |
| | 9.9 | 9.2 | 16.6 |
| | 10.0 | 9.1 | 16.7 |
| | 10.0 | 9.1 | 16.7 |
| | 10.0 | 9.2 | 16.7 |
| | 10.0 | 9.1 | 16.8 |
| | 9.9 | 9.2 | 16.8 |
| | 9.9 | 9.2 | 16.7 |
| | 9.8 | 9.2 | 16.7 |
| | 9.8 | 9.2 | 16.7 |
| Mean value | 9.92 | 9.17 | 16.69 |
| Coefficient of variation | 0.80% | 0.53% | 0.52% |

Table 2

| | Added concentration (mg/dl) | | | |
|---|---|---|---|---|
| | 0 | 5 | 10 | 30 |
| Measured value | 7.1 | 12.0 | 17.2 | 37.4 |
| Recovery concentration | - | 4.9 | 10.1 | 30.3 |
| Recovery rate (%) | - | 98.0 | 101.0 | 101.0 |

Test Example 6 (Correlation with conventional method)

A quantitative measurement of uric acid on each of 48 samples was carried out in accordance with the

invention method as described in Example 2 and the conventional 4-aminoantipyrine-phenol method, respectively to check the correlation therebetween. Results are shown in Fig. 7. Both methods show good correlation, with a coefficient of correlation r = 0.9998 and a correlative formula y = 0.998x - 0.03. This shows the reliability of the method of the invention.

## Claims

1. A method of quantitatively measuring hydrogen peroxide in a sample, which comprises adding to the sample: (a) a phenol or naphthol compound of the formula

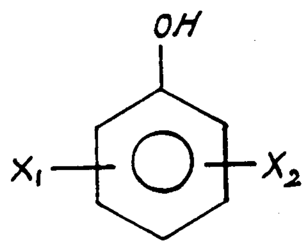

or

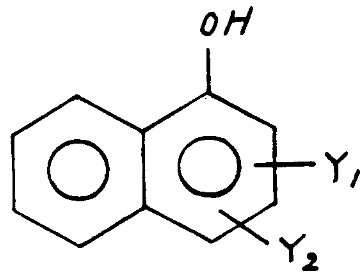

wherein $X_1$ and $X_2$ are each a hydrogen atom, halogen atom, alkyl group or nitro radical, and $Y_1$ and $Y_2$ are each a hydrogen atom, halogen atom, nitro radical, sulfo radical or acyl group, (b) p-diethylaminoaniline and (c) an oxidase, to cause a reaction and form a quinonic dye, and measuring the intensity of the color of the resulting solution to determine the amount of the hydrogen peroxide.

2. A method as claimed in Claim 1, wherein said oxidase is peroxidase.

3. A method of quantitatively measuring biochemical substances and enzymes in a sample obtained from a living body, which comprises reacting the sample with the enzyme to liberate hydrogen peroxide, adding the reagents (a), (b) and (c) as defined in Claim 1 or 2 to cause a reaction with the liberated hydrogen peroxide and form a quinonic dye, and measuring the color tone of the resulting solution to determine the amount of biochemical substance or enzyme in the sample.

4. A method as claimed in Claim 3, wherein the sample is of serum from blood.

5. A method as claimed in Claim 3 or 4, wherein the substance measured in the sample is uric acid.

6. A method as claimed in any preceding claim, wherein the phenol or naphthol compound (a) is phenol, p-hydroxybenzoic acid, p-chlorophenol, 2,4-dichlorophenol, 4,6-dichloro-o-cresol, 2,4-dibromophenol, 4,6-dibromo-o-cresol, α-naphthol or 1-naphthol-2-sulfonic acid.

## Revendications

1. Méthode pour mesurer quantitativement le peroxyde d'hydrogène dans un échantillon, qui comprend les étapes consistant à ajouter à l'échantillon: a) un composé du phénol ou du naphthol de la formule

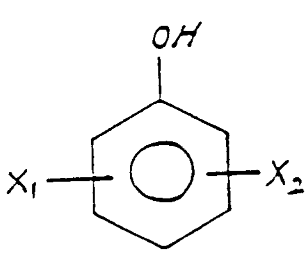

ou

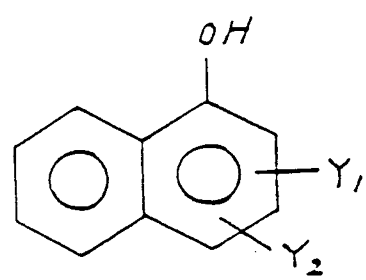

dans laquelle $X_1$ et $X_2$ sont chacun un atome d'hydrogène, un atome d'halogène, un groupe alkyle ou un radical nitro, et $Y_1$ et $Y_2$ sont chacun un atome d'hydrogène, un atome d'halogène, un radical nitro, un radical sulfo ou un groupe acyle, (b) de la p-diéthylaminoaniline et (c) un oxydase, pour provoquer une réaction et former un colorant quinonique, et à mesurer l'intensité de couleur de la solution qui en résulte afin de déterminer la teneur en peroxyde d'hydrogène.

**2.** Méthode selon la revendication 1, dans laquelle le dit oxydase et le péroxydase.

**3.** Méthode pour mesurer quantitativement des substances biochimiques et des enzymes dans un échantillon obtenu à partir d'un corps vivant, comprenant les étapes consistant à mettre en réaction l'échantillon avec l'enzyme pour libérer le peroxyde d'hydrogène, à ajouter les réactants (a), (b) et (c) selon la revendication 1 ou la revendication 2 pour provoquer une réaction avec le peroxyde d'hydrogène libéré et former un colorant quinonique, et à mesurer la note de couleur de la solution qui en résulte afin de déterminer la teneur en substance biochimique ou en enzyme de l'échantillon.

**4.** Méthode selon la revendication 3, dans laquelle l'échantillon est du sérum sanguin.

**5.** Méthode selon la revendication 3 ou la revendication 4, dans laquelle la substance mesurée dans l'échantillon est de l'acide urique.

**6.** Méthode selon l'une quelconque des revendications précédentes, dans laquelle le composé du phénol ou du naphthol (a) est le phénol, l'acide p-hydroxybenzoïque, le p-chlorophénol, le 2,4-dichlorophénol, le 4,6-dichloro-o-crésol, le 2,4-dibromophénol, le 4,6-dibromo-o-crésol, le α-naphthol ou l'acide 1-naphthol-2-sulfonique.

**Patentansprüche**

**1.** Verfahren zur quantitativen Messung von Wasserstoffperoxid in einer Probe, umfassend die Zugabe zu einer Probe von:

(a) einer Phenol- oder Naphtholverbindung der Formel

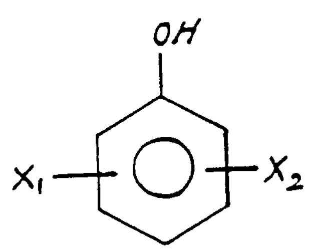

oder

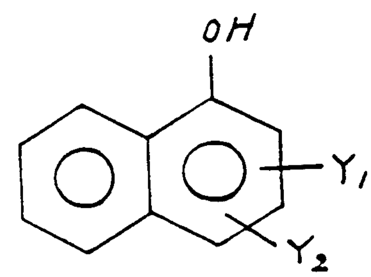

worin $X_1$ und $X_2$ je ein Wasserstoffatom, ein Halogenatom, eine Alkylgruppe oder eine Nitrogruppe sind und $Y_1$ und $Y_2$ je ein Wasserstoffatom, ein Halogenatom, ein Nitrorest oder ein Sulforest oder eine Acylgruppe sind,

(b) p-Diethylaminoanilin und (c) eine Oxidase, um eine Reaktion zu bewirken und einen Chinonfarbstoff zu bilden und Messung der Farbintensität der resultierenden Lösung um die Menge des Wasserstoffperoxids zu bestimmen.

**2.** Verfahren gemäss Anspruch 1, worin die genannte Oxidase Peroxidase ist.

**3.** Verfahren zur quantitativen Messung von biochemischen Substanzen und Enzymen in einer Probe, welche aus dem lebenden Körper erhalten wird, umfassend die Umsetzung der Probe mit einem Enzym um Wasserstoffperoxid freizusetzen, Zugabe der Reagenzien (a), (b) und (c) wie in Anspruch 1 oder 2 definiert, um eine Reaktion mit dem freigesetzten Wasserstoffperoxid zu bewirken und Bildung eines Chinonfarbstoffes und Messen des Farbtones der resultierenden Lösung, um die Länge der biologischen Substanz oder des Enzyms in der Probe zu bestimmen.

**4.** Verfahren gemäss Anspruch 3, worin die Probe Serum aus Blut ist.

**5.** Verfahren gemäss Anspruch 3 oder 4, worin die in der Probe gemessene Substanz Harnsäure ist.

**6.** Verfahren gemäss einem der vorhergehenden Anspruche, worin die Phenol- oder Naphtholverbindung (a) Phenol, p-Hydroxybenzoesäure, p-Chlorphenol, 2,4-Bichlorphenol, 4,6-Dichlor-o-kresol, 2,4-Dibromphenol, 4,6-Dibrom-o-kresol, α-Naphthol oder 1-Naphthol-2-sulfonsäure ist.

FIG. 1

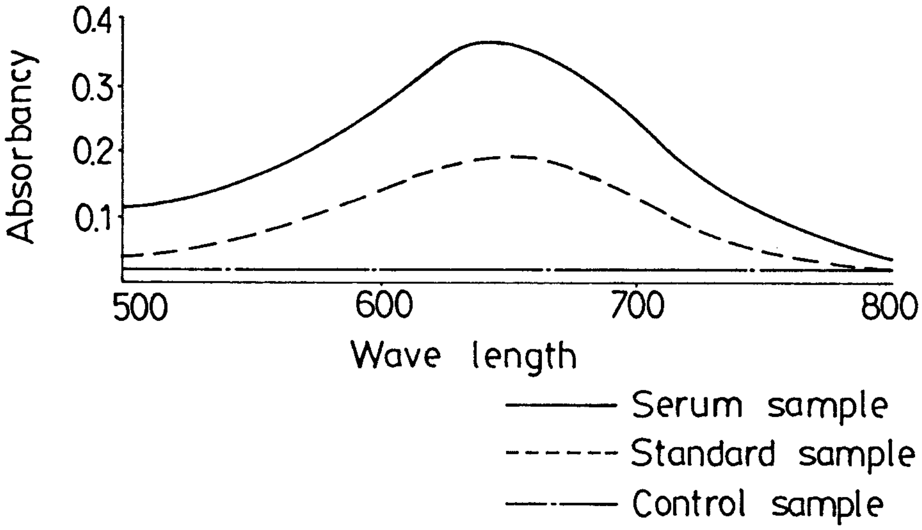


FIG. 2

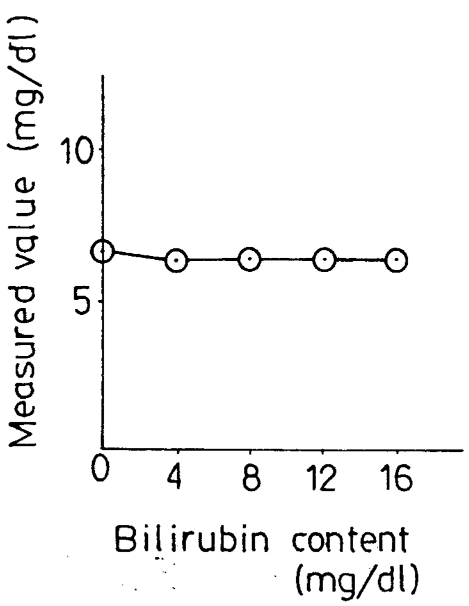


FIG. 3

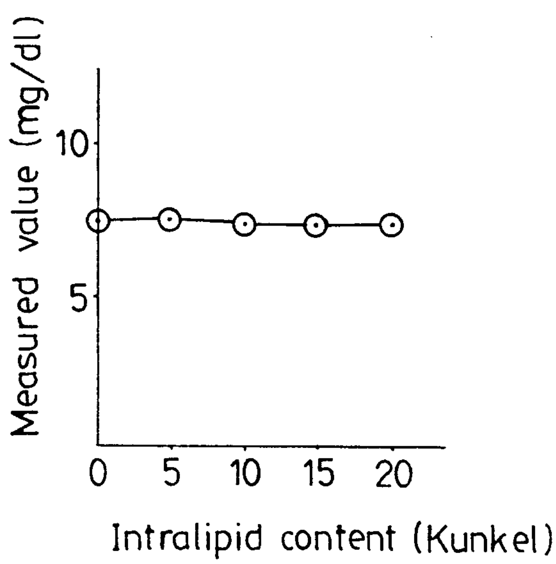

FIG. 4

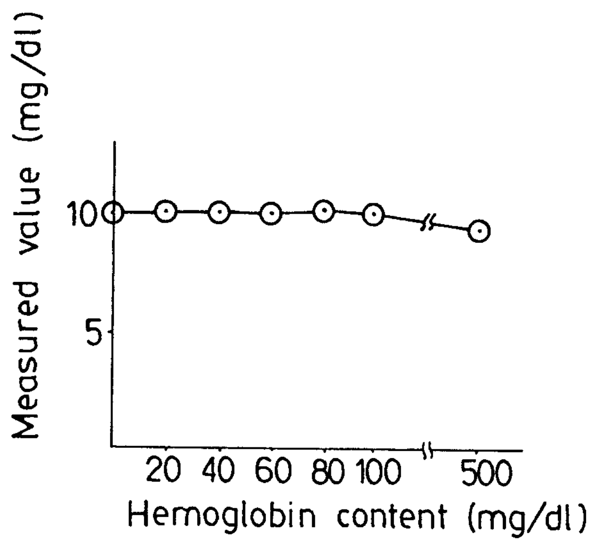
Measured value (mg/dl)
10
5
20 40 60 80 100 500
Hemoglobin content (mg/dl)

FIG. 5

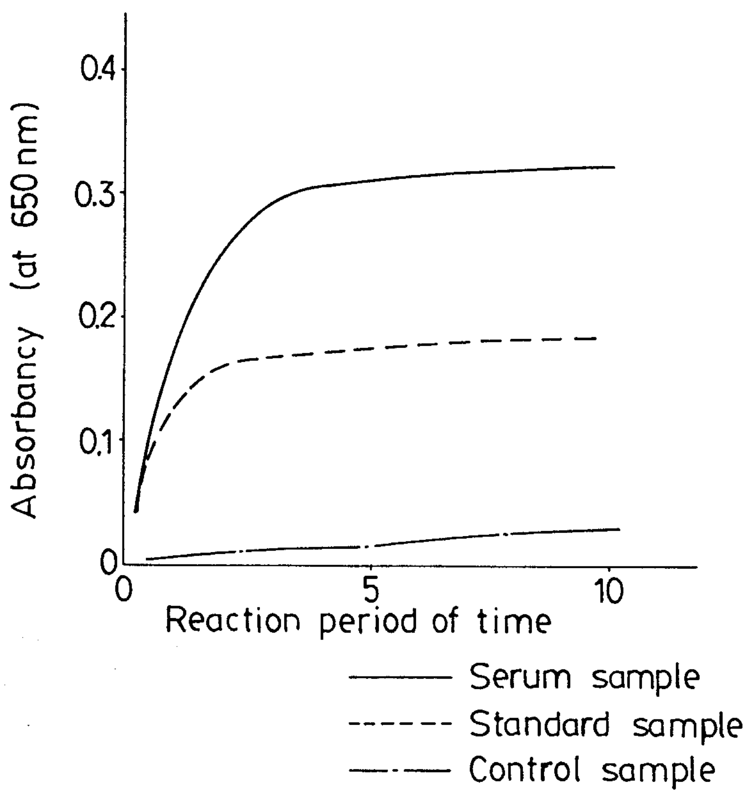
Absorbancy (at 650 nm)
0.4
0.3
0.2
0.1
0
0
5
10
Reaction period of time
Serum sample
Standard sample
Control sample

FIG. 6

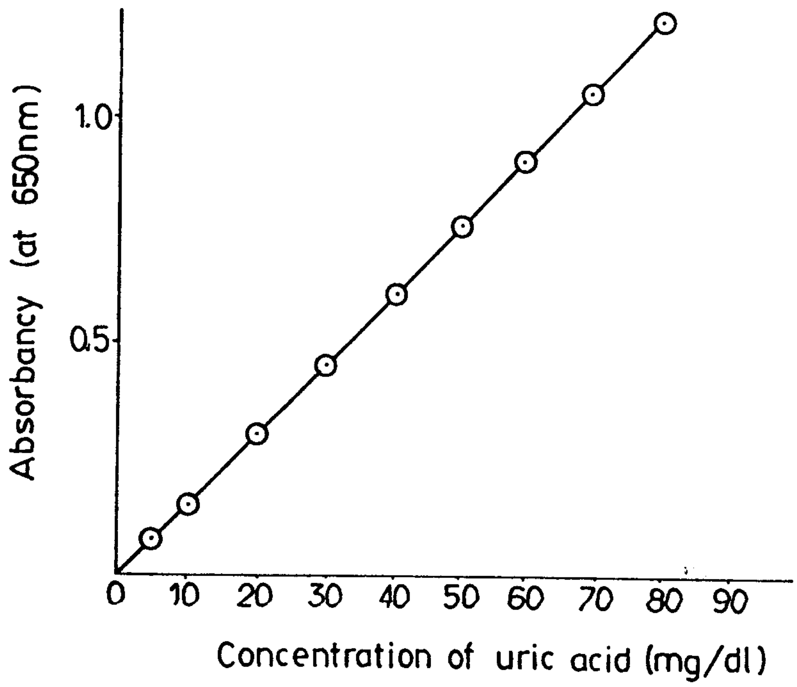
Absorbancy (at 650nm)
1.0
0.5
0
10
20
30
40
50
60
70
80
90
Concentration of uric acid (mg/dl)

FIG. 7

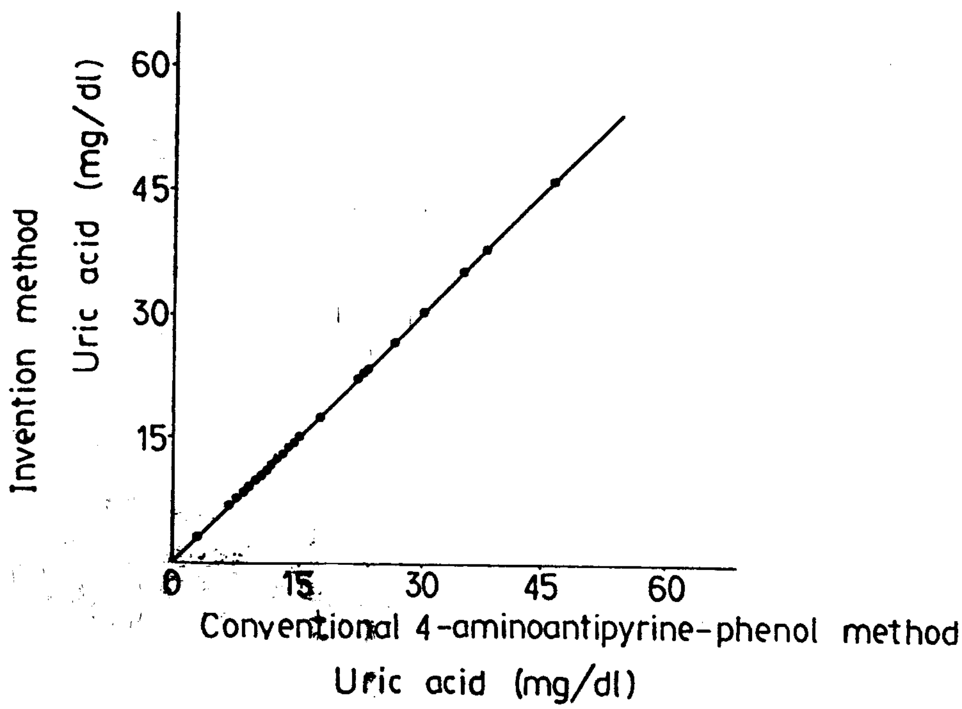
Invention method
Uric acid (mg/dl)
60
45
30
15
0
15
30
45
60
Conventional 4-aminoantipyrine-phenol method
Uric acid (mg/dl)